# EUROPEAN PATENT SPECIFICATION

(11) **EP 2 121 026 B1**
(45) Date of publication and mention of the grant of the patent: **28.06.2017**
(21) Application number: 07849557.9
(22) Date of filing: 11.12.2007
(51) Int. Cl.: A61K 47/36, A61K 9/06

(54) **NOVEL INJECTABLE CHITOSAN MIXTURES FORMING HYDROGELS**
NEUE INJIZIERBARE HYDROGEL BILDENDE CHITOSAN-GEMISCHE
NOUVEAUX MÉLANGES INJECTABLES DE CHITOSANS FORMANT DES HYDROGELS

(30) Priority: 11.12.2006 US 873931 P; 21.05.2007 US 924582 P
(43) Date of publication of application: 25.11.2009
(73) Proprietor: CHIT2GEL Ltd., 69710 Tel Aviv (IL)
(72) Inventor: BEN-SHALOM, Noah, 69278 Tel-aviv (IL); NEVO, Zvi, 46412 Herzlia (IL); PATCHORNIK, Abraham, 70400 Ness-ziona (IL); ROBINSON, Dror, 99788 Kefar Shmuel 100 (IL)
(74) Representative: Becker Kurig Straus
(86) International application number: PCT/IL2007/001530
(87) International publication number: WO 2008/072230

(56) References cited:
- WO-A-01/10421
- WO-A-99/07416
- WO-A-03/011912
- WO-A-2004/068971
- WO-A-2004/069230

## Description

### FIELD OF THE INVENTION

The present invention relates to the field of positively charged polysaccharide hydrogels. More particularly, the present invention relates to pH dependent, thermosensitive polysaccharide hydrogels, comprising a composition of at least two different chitosans differing in the degree and ratio of acetylation, the homogenic or non-homogenic distribution of the acetylated sites, their molecular weights, solubility, and biodegradability, and methods of preparation thereof.

### BACKGROUND OF THE INVENTION

Hydrogels are highly hydrated, macromolecular networks, dispersed in water or other biological fluids.

Hydrogels that exhibit the specific property of increased viscosity with increased temperatures are known as thermosensitive (or thermosetting) hydrogels. Such hydrogels have been shown to have easier application combined with longer survival periods at the site of application as compared to non-thermosensitive hydrogels, and are therefore advantageous as slow-release drug delivery systems.

It is known that thermosensitive hydrogels may be prepared from polymers of natural origin (O. Felt et al. in The Encyclopedia of Controlled Drug Delivery, 1999), such as chitosan, which is a commercially available, inexpensive polymer obtained by partial to substantial alkaline N-deacetylation of chitin, a linear polysaccharide, made of N-acetylglucosamine units, linked via β-1,4 glycosidic bonds. The deacetylation process is generally performed using hot, concentrated, hydroxide solutions, usually sodium hydroxide.

Chitin is a naturally occurring biopolymer, found in the cytoskeleton and hard shells of marine organisms such as crustacea, shrimps, crabs, fungi, etc., and is the third most abundant naturally occurring polysaccharide after cellulose and laminarine. Chitin is chemically inert, insoluble and, has a crystalline structure in the form of flakes, crumbs or tiles.

Chitosan contains free amine (-NH₂) groups and may be characterized by the proportion of N-acetyl-D-glucosamine units to D-glucosamine units, commonly expressed as the degree of acetylation (DA) (reciprocal to deacetylation) of the chitin polymer. Both the degree of acetylation, and the molecular weight (MW) are important parameters of chitosan, influencing properties such as solubility, biodegradability and viscosity.

Chitosan is the only positively charged polysaccharide, making it bioadhesive, which delays the release of a medication agent from the site of application (He et. al., 1998; Calvo et. al., 1997), and allows ionic salt interactions with anionic natural compounds such as glyosaminoglycans of the extra-cellular membrane.

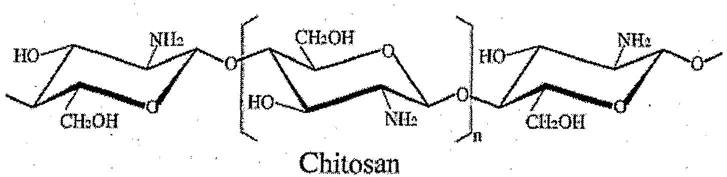

Chitosan is biocompatible, non-toxic, and non-immunogenic, allowing its use in the medical, pharmaceutical, cosmetic and tissue construction fields. For example, topical ocular applications and intraocular injections or transplantation in the vicinity of the retina (Felt et. al., 1999; Patashnik et. al.; 1997; Song et. al., 2001). Moreover, chitosan is metabolized-cleaved by certain specific enzymes, e.g. lysozyme, and can therefore be considered as bioerodable and biodegradable (Muzzarelli 1997, Koga 1998). In addition, it has been reported that chitosan acts as a penetration enhancer by opening epithelial tight-junctions (Junginger and Verhoef, 1998; Kotze et. al., 1999), similar to the action of the enzyme hyaluronidase, the so called "spreading factor". Chitosan also promotes wound-healing, as well as acting as an antiadhesive (preventing pathological adhesions) (Biagini et. al., 1992; Ueno et. al., 2001) and exhibits anti-bacterial, (Felt et. al., 2000; Liu et. al., 2001), anti-fungal effects, and anti-tumor properties.

Considering the remarkable properties of chitosan, there is a growing need for new chitosan hydrogels for use in the growing industries of slow-release of drugs and regenerative medicine.

Recently, temperature-controlled pH-dependant formation of ionic polysaccharide gels, such as chitosan/organo-phosphate aqueous systems, has been described (US Patent No. 6,344,488). While chitosan aqueous solutions are pH-dependant gelating systems, the addition of a mono-phosphate dibasic salt of polyol or sugar to a chitosan aqueous solutions leads to further temperature-controlled pH-dependant gelation. Solid organo-phosphate salts are added and dissolved at low temperature within 0.5 to 4.0% w/v chitosan in aqueous acidic solutions. Aqueous chitosan/organo-phosphate solutions are initially stored at low temperatures (4°C), then endothermally gelated within the temperature range of 30 to 60°C. Chitosan/organo-phosphate solutions rapidly turn into gels at the desired gelation temperature.

Multiple interactions are responsible for the solution/gel transition: The increase of chitosan interchain hydrogen bonding, as a consequence of the reduction of electrostatic repulsion, due to the basic nature and action of the salt, and the chitosan-chitosan hydrophobic interactions which should be enhanced by raising the pH. The gelation process that occurs upon increasing the temperature, predominantly originates due to the strengthening of the chitosan hydrophobic attractions, also shown in the presence of the glycerol moiety (serving as a plasticizer) and chitosan. At low temperatures, strong chitosan-water interactions, protects the chitosan chains from aggregation. Upon heating, sheaths of water molecules are removed, allowing the association of aligned chitosan macromolecules. Furthermore, electrostatic forces may decrease upon raising the temperature, and the hydrophobic interactions are expected to have a major contribution to the gelation of the chitosans mixture.

Transparent chitosan/glycerophosphate hydrogels have been prepared, requiring modification of deacetylation of chitosan by reacetylation with acetic anhydride. The use of previously filtered chitosan, dilution of acetic anhydride and reduction of temperature has been shown to improve efficiency and reproducibility (Berger et al., 2004). Turbidity of chitosan/glycerophosphate hydrogels has been shown to be modulated by the degree of deacetylation of chitosan and by the homogeneity of the medium during reacetylation, which influences the distribution mode of the glucose amine monomers. The preparation of transparent chitosan/glycerophosphate hydrogels requires a homogeneously reacetylated chitosan with a degree of deacetylation between 30 and 60%.

Hydrogels comprising chitosan are very useful for drug delivery. They may conveniently be administered by local (intra-articular) routes; they are injectable using minimally invasive procedures; drug delivery using hydrogels provides a high level of concentration of the drug directly at the target site; and they minimize adverse systemic effects and toxicity of the drug.

Chitosan microspheres have been developed for the delivery of drugs, in which drug release is controlled by particle size, degree of hydration, swelling ratio or biodegradability of the prepared microspheres. Attempts have been made to develop chitosan microspheres for the delivery of drugs such as anti-cancer drugs, peptides, antibiotic agents, steroids, etc. by cross-linking of chitosan to form a network.

Conventional chitosan cross-linking reactions have involved a reaction of chitosan with dialdehydes, which may have physiological toxicity. Novel chitosan networks with lower cytotoxicity were synthesized using a naturally occurring crosslinker called genipin, which provides bifunctional crosslinking by heterocyclic linking of genipin with chitosan by a nucleophilic attack and the formation of amide linkages (Mi et al., 2000).

The preparation of thermosensitive, neutral solutions based on chitosan/polyol salt combinations has been described by Chenite et al., 2000. These formulations possess a physiological pH and can be held liquid below room temperature for encapsulating living cells and therapeutic proteins; they form monolithic gels at body temperature, without any chemical modification or cross-linking. The addition of polyol salts bearing a single anionic head results in the formation of a gel due to synergistic forces favorable to gel formation, such as hydrogen bonding, electrostatic interactions and hydrophobic interactions. When injected *in vivo* the liquid formulation turns into gel implants *in situ.* The system has been used as a container-reservoir for delivery of biologically active growth factors *in vivo* as well as an encapsulating matrix for living chondrocytes for tissue engineering applications.

Chitosan-glycerol phosphate/blood implants have been shown to improve hyaline cartilage repair in microfacture defects by increasing the amount of tissue and improving its biochemical composition and cellular organization (Hoemann et al., 2005). The microfracture defect is filled with a blood clot inhabited by bone-marrow derived cells, that has been stabilized by the incorporation of chitosan. The use of such implants would therefore be expected to result in better integration, improved biochemical properties, and longer durability of the repair tissue.

Uniform submicron chitosan fibers, which may have an important application as artificial muscles, as biosensors, or as artificial organ components, may be prepared by electro-wet-spinning technology (Lee et al. 2006).

Chitosan-based gels have been shown to turn into and serve as scaffolds for the encapsulation of invertebral disc (IVD) cells (Roughley et al., 2006), by entrapping large quantities of newly synthesized anionic proteoglycan. Such gels would therefore be a suitable scaffold for cell-based supplementation to help restore the function of the nucleus pulposus structural region during the early stages of IVD degeneration. A denser, fibrillar collagen fabric may serve as an annulus fibrosis structural substitute, allowing colonization with endogenous cells.

Collagen gel has previously been shown to be useful for repair of articular cartilage defects with cultured chondrocytes embedded in the gel (Katusbe et al., 2000). More recently, chitosan hydrogels have been shown to be useful for cartilage regeneration and prevention of knee pain associated with acute and chronic cartilage defects.

An advanced clinical product of such chitosan hydrogels is a hydrogel produced by BioSyntech, described in PCT application WO 99/07416. The thermosensitive chitosan hydrogel of BioSyntech is prepared by neutralizing a commercial chitosan, having a degree of deacetylation of about 80-90%, with mono-phosphate dibasic salts of polyols, particularly β-glycerophosphate (β-GP). Addition of β-GP to chitosan enables the pH to be increased up to about 7 without chitosan precipitation, and to form a hydrogel at that pH, at physiological temperature.

A chitosan hydrogel (BST-CarGel™) is produced by BioSyntech, which fills cartilage defects and provides an optimal environment for cartilage repair. The chitosan plasticizer mixture is delivered within a debrided cartilage defect following microfracture, using the patient's own blood as a sole source of biological ingredients. The mixture fills the defect and solidifies in situ within 8-12 minutes, providing an effective scaffold for cartilage regeneration. Healthy chondrocytes then migrate from the deep inner bone through the microfracture pores and repopulate the gel-filled lesion.

A second BioSyntech chitosan hydrogel, BST-DermOn™, may be used as a topical therapy for stimulating and supporting wound healing. The product acts as a mucoadhesive barrier and can seal the wound and maintain a moist environment while continuing to allow gas exchange.

A further BioSyntech chitosan hydrogel, BST-InPod™, is intended for treatment of heel pain. This is an injectable product which is intended to permanently restore comfort of plantar fat pads by integrating with the patient's own pad fat and restoring biomechanical cushioning properties and comfort.

These promising examples also exhibit some limitations. The BioSyntech products comprising commercially available chitosan, having a degree of acetylation, of about 15-20% DA, are believed to exert an undesired slower degradation rate. Futhermore, chitosan has limited ability to mix with and encapsulate cells at physiological pH of 7.4 to form a three-dimensional scaffold.

The BioSynthech family of hydrogels have limited degradation rates and the formation of such hydrogels require the presence of glycerophosphate or similar plasticizing salts. Glyerophosphate is a negatively charged molecular entity that can react with positive charges of bioactive components, leading to their precipitation, or to the disturbance of their release from the hydrogel. Therefore, the presence of glycerophosphate may decrease the range of drugs with which chitosan/glyceroposphate hydrogels can be used.

Further, the modulation of the properties of the hydrogel, such as gelation time and viscosity, depends on the concentration of glycerophosphate, and is therefore limited by the solubility of glycerophosphate. In particular, a high concentration of glycerophosphate is required to have a low gelation time, avoiding the rapid elimination of the hydrogel after its administration. However, a high concentration of glycerophosphate also decreases the viscosity of the hydrogel. Therefore, the gelation time has to be balanced with the consistency of the hydrogel, and it is not possible to obtain hydrogels that have both low gelation time and high viscosity, which would be a desirable combination of characteristics. Also, a too high concentration of glycerophosphate may induce the precipitation of the hydrogel at its administration site.

Further, thermosensitive chitosan/glycerophoshpate gels were found to be turbid, thus rendering their use inappropriate for particular applications such as ocular or topical adminstrations.

A novel type of chitosan has been identified. It has been found that reacetylation of commercial chitosan to produce homogenously acetylated chitosans having a degree of acetylation of from about 30% to about 60%, greatly increases the solubility of the chitosan in water and body fluids at physiological pH, without the need to use glycerol phosphate. Such chitosans produce clear transparent gels, which may be used for cell encapsulation (WO 05/097871 to Berger et al).

An example of commercial chitosan which may be used in the preparation of reacetlyated chitosan is a chitosan of pharmaceutical grade and high MW obtained from Aldrich Chemical, Milwaukee, USA, having a MW of 1100 kDa as determined by size exclusion chromatographic method reported by O. Felt, et al. in Int. J. Pharm. 180, 185-193 (1999) and a deacetylation degree DD of 83.2 % as measured by UV method reported by R.A. Muzarelli et al. in "Chitin in Nature and Technology", Plenum Press, New York, 385- 388, (1986). However, any commercial chitosan having a deacetylation degree of 80 to 90 % and a molecular weight not smaller than 10 kDa may be used. The acidic medium used for dissolving commercial chitosan may be for example acetic acid and the acidic solution of chitosan obtained after solubilization of chitosan may be then diluted with an alcohol, for example methanol.

Homogeneous reacetylation of chitosan on one hand has the effect of increasing the number of hydrophobic sites by replacing amine groups with acetyl groups, but on the other hand the crystalline structure that makes chitosan tend to fold is highly reduced cumulating in increased solubility of the chitosan. Reacetylation prevents refolding of the polymer, maintaining the straight chain, and thus preventing the pH-related decrease in solubility.

Generally, commercially available chitosan is industrially prepared by deacetylation of dry chitin flakes (Muzzarelli, 1986). Deacetylation preferentially occurs in the amorphous zones of the chitin molecules at the surface of the flakes, resulting in non-homogeneous monomers with variable block size of deacetylated-units distribution (Aiba, 1991). In comparison, reacetylated chitosan under homogeneous conditions, adopts a random distribution of deacetylated monomers, which induces a decrease of the crystallinity of chitosan and in turn increase its solubility (Aiba, 1991, 1994; Ogawa and Yui, 1993; Milot et. al., 1998). WO 2004/069230 relates to pharmaceutical compositions comprising inter alia a release sustaining or mucoadhesive agent in form of chitosans with a degree of acetylation ranging from 25 to 80%. If more than one chitosan is used, the other may a degree of acetylation of below 25%, i.e. a degree of deacetylation of above 75%. WO 2004/069230 does not disclose a negatively charged polysaccharide.

The suitability of polymeric hydrogels for an application is dictated by their biocompatibility, mechanical integrity, speed and reversibility of gel formation at physiogical pH, and their low weight and extended lifetime. However, very little control is possible over various important properties of known chitosan hydrogels, such as strength, rate of degradation, and release profile.

There is thus a widely recognized need for, and it would be highly advantageous to have a hydrogel comprising chitosans, wherein physical and properties of the gel can be manipulated as required.

### SUMMARY OF THE INVENTION

The present invention provides a pH- and temperature-dependent composition for formation of a polysaccharide hydrogel.

According to one aspect of the present invention there is provided a chitosan composition comprising at least one type of chitosan having a degree of acetylation in the range of from about 30% to about 60%, and at least one type of chitosan having a degree of deacetylation of at least about 70%, wherein the composition further comprises a negatively charged polysaccharide, and wherein the composition forms a hydrogel at near physiological pH at 37°C. According to another aspect of the present invention there is provided a method for the production of a stable hydrogel comprising a composition of at least one highly acetylated chitosan having a degree of acetylation of from about 30 to about 60%, and at least one highly deacetylated chitosan having a degree of deactylation of from about 70%, wherein the composition further comprises a negatively charged polysaccharide. The method comprises dissolving at least one highly acetylated chitosan and at least one highly deacetylated chitosan in acid to form a composite solution; adjusting the pH of the composite solution to a value of from above 6.5 to about 7.2; and increasing the temperature of the composite solution to about 37°C while raising the pH to from about 7.0 to about 7.6.

According to yet another aspect of the present invention, there is provided a slow release drug delivery system comprising a hydrogel comprising a composition of at least one chitosan having a degree of acetylation of from about 30 to about 60%, and at least one highly deacetylated chitosan having a degree of deactylation of from about 70%, wherein the composition further comprises a negatively charged polysaccharide. The chitosan gel resulting from this mixture of at least two chitosan types may optionally comprise microspheres of chitosan encapsulating a drug and/or electrospun chitosan fibers embedded in the gel.

According to further features in preferred embodiments of the invention described below, gelation of the composition occurs at a pH higher than pH 6.5.

The highly acetylated chitosan may be either homogenously acetylated or homogenously deacetylated. Optionally and preferably, the highly deacetylated chitosan is non-homogenously deacetylated.

The highly acetylated chitosan and the highly deacetylated chitosan may optionally each be present at a concentration of from about 0.1% to about 6 % w/v of the total composition, and may each have a molecular weight in the range of from about 10 kDa to about 4000 kDa. Optionally and preferably, the highly deacetylated chitosan has a molecular weight of greater than about 200 kDa. Further optionally and preferably, the highly acetylated chitosan has a molecular weight of greater than about 60 kDa.

The properties of the hydrogel may be controlled by manipulation of the molecular weight, degree of deacetylation and distribution of the deacetylated sites of both the highly acetylated chitosan and the highly deacetylated chitosan. These manipulations will influence the gel properties, such as, for example, the gelation temperature, density or porosity, or the degree of hydration or the degree of hydrophobicity. The degradation rate of the hydrogel may be further controlled by binding of the lysozyme inhibitor Tri-N-acetyl-glucosamine to the highly acetylated chitosan.

The composition of the present invention comprises a negatively charged polysaccharide, such as, for example, an animal- or plant-derived polymer. As a non-limiting example of a plant-derived polymer, the negatively charged polysaccharide may optionally comprise seaweed. Alternatively, the negatively charged polysaccharide may optionally comprise a gycosaminoglycan, such as, for example, hyaluronic acid, chondroitin sulfate, or other acidic polymers such as dextran sulphate.

According to further features in the preferred embodiments, the composition of the present invention may further comprise at least one of a drug, a polypeptide and a cell (such as an animal cell or a plant cell).

The composition may further comprise an emulsifier. Optionally, the chitosans and the emulsifier may form nanoparticles.

The hydrogel of the present invention may optionally be used in an application such as, for example, drug delivery, support of cell growth, bone structural support, cartilage repair, tissue reconstruction, wound- healing, production of artificial skin, as a hypolipidemic and hypocholesterolimic agent, formation of artificial kidney membrane, bone filling, and soft tissue reconstruction as for heel pain relief for example. The composition for formation of the hydrogel optionally be administered by a route such as injection or endoscopic administration.

The hydrogel may optionally be used in the preparation of a biocompatible material for use in the preparation of an implantable device, such as for use in tissue repair, tissue reconstruction, tissue construction, and tissue replacement.

In some embodiments, the anti-adhesion properties of chitosan makes this gel useful as an anti adhesion device in applications such as cardio thoracic surgery and abdominal surgery for example.

According to further features in the preferred embodiments, the hydrogel may optionally be used in the preparation of a drug delivery device. The drug delivery device may optionally provide slow release of an embedded medication. Non-limiting examples of drugs for use in this system include proteins (such as BSA or hemoglobin) or non-protein agents (such as, for example, ACE-inhibitors or anti inflammatory drugs). The drug delivery system may optionally be an opthalmological drug delivery system due to the transparency of the gel. However the drug delivery system may also optionally be implemented for urological applications such as vesicoureteral reflux and in cosmetic applications as for example wrinkle treatment for example.

The drug may also optionally comprise one or more of a mineral, a vitamin, a food additive or natural extract such as a plant derived extract for example. The gel itself, optionally with an active ingredient, may optionally be used as a food additive.

Alternatively, the hydrogel may optionally be used for supporting endogenous cells in a three-dimensional gel construct. As a further alternative, the hydrogel may optionally be used for embedding exogenous cells with or without added growth factors, as well the cell may provide metabolites such as growth factors. Also alternatively, the hydrogel may optionally be used in the production of a cell-loaded artificial matrix, where the cells are, for example, chondrocytes, fibrochondrocytes, ligament fibroblasts, skin fibroblasts, tenocytes, myofibroblasts, mesenchymal stem cells and keratinocytes.

According to preferred embodiments of the present invention, there is provided a chitosan composition comprising nanoparticles containing an active ingredient and encapsulated in a hydrogel comprising at least one type of chitosan having a degree of acetylation in the range of from about 30% to about 60%, and at least one type of chitosan having a degree of deacetylation of at least about 70%, wherein the composition further comprises a negatively charged polysaccharide, wherein the hydrogel is formed through pH- and temperature-dependant gelation. Optionally, the composition further comprises an emulsifier. Also optionally, the hydrogel forms upon injection to a subject.

According to some embodiments, chitosan gel may optionally be used as a lubricating agent in such applications such as vaginal atrophy, dry eyes, conjuctivitis sicca, dry nose following upper respiratory infections as well as a general soothing agent for various abrasions. Chitosan gel may also optionally be used as an anti-inflammatory agent in fascial diseases such as fibromyalgia by either local injection or external massage.

The present invention successfully addresses the shortcomings of the presently known compositions for formation of polysaccharide hydrogels by providing a composition which forms a hydrogel in which the physical and chemical properties can be accurately determined.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the figures:
FIG. 1 illustrates the formation of a hydrogel according to the present invention from a liquid composition comprising two different types of chitosan; and
FIG. 2a illustrates degradation times of a composition comprising chitosan type 1 and type 2 in accordance with the principles of the present invention;
FIG. 2b illustrates degradation times of a composition comprising chitosan type 1 and type 2 at different ratios;
FIG. 3 illustrates release of hemoglobin from the hydrogel of the present invention as measured by µg/ml in eluent;
FIG. 4 illustrates release of bovine serum albumin (BSA) from the hydrogel of the present invention as measured by optical density (OD);
FIG. 5 presents a bar chart illustrating release of BSA from the hydrogel of the present invention;
FIG. 6 illustrates the degradation profile of the hydrogel of the present invention;
FIG. 7 illustrates the integration of the release profile of BSA with the degradation profile of the hydrogel of the present invention;
FIGS. 8A and 8B show histopathology of wound bed biopsies taken from rats treated with the hydrogel of the present invention;
FIG. 9 shows a graph of the results of treatment; and
FIGS. 10A and 10B show the results of in vivo experiments performed on rats for rotator cuff damage.

### DESCRIPTION OF THE PREFERRED EMBODIMENTS

The present inventors have found that a composition comprising at least two different types of chitosans, wherein the different types are classified according to their degree of acetylation/deacetylation and the level of homogeneity of the acetylated units, provides a hydrogel in which a greater degree of control over various physical and chemical properties is possible, as compared to hydrogels comprising a single type of chitosan.

Chitosans which are deacetylated to a degree of deactylation (DD) of about 70-100% (i.e. DA of up to about 30%), such as commercially available chitosan, may be termed Type 1 chitosans. These chitosans are insoluble at physiological pH, and are poorly recognized by lysozyme, resulting in slow biodegradation. Gels formed by chitosans of this type have a low degree of acetylation, forming dense hydrogen bonds with many hydrophobic interactions. The degradation rate has been shown to be a function of the degree of deacetylation. Degradation of chitosan has an influence on cell proliferation and remodeling.

Highly homogeneously deacetylated or reacetylated chitosans (having degree of acetylation of from about 30% to about 60%) are termed type 2. Such chitosans are readily digested/degraded by lysozyme, thereby enabling controlled drug release. If deacetylation degree of chitosan is lower than 30 %, the chitosan becomes a polymer close to chitin that is insoluble in acidic conditions and consequently not usable in the present invention. At a degree of deacetylation greater than 60 %, precipitation of chitosan occurs.

The deacetylation degree of chitosan may be determined by a spectrophotometric method such as described in the literature by R.A. Muzarelli and R. Richetti in Carbohydr. Polym. 5, 461-472, 1985 or R.A. Muzarelli and R. Richetti in "Chitin in Nature and Technology", Plenum Press 385-388, 1986. Briefly, in the latter method for example, chitosan is solubilized in 1 % acetic acid and the DD is determined by measuring its content of N-acetyl-glucosamine by UV at 200, 201, 202, 203 and 204 nm using N-acetyl-D-glucosamine solutions as standards The present invention relates to a polysaccharide hydrogel composition comprising a combination of at least one highly acetylated chitosan having a degree of acetylation of from about 30% to about 60%, and at least one highly deacetylated chitosan, having a degree of deacetylation of at least about 70%.

The highly acetylated type 2 chitosans can interact through electrostatic, hydrogen and hydrophobic interactions with the highly deacetylated chitosans of family type 1. The extent of interaction increases with increasing pH. A composition comprising solutions of both types of chitosan can form a stable gel at physiological pH, without the need for glycerophosphate.

Type 1 chitosans precipitate at a pH of about 6.5, which is less than physiological pH. Interaction of the highly hydrophobic, homogenous, chitosan type 2 with chitosan type 1 prevents this precipitation of the non-homogenously acetylated type 1 chitosan, by formation of hydrogen and hydrophobic bonds, allowing the formation of a stable solution from about pH 6.7, and a stable semi-solid hydrogel at about pH 7.0 and above.

The secondary bonds which are formed allow the encapsulation of the non-homogenous chitosan chains and maintain it solubility at pH greater than its pKa value. Generally, such secondary chain interactions are the main molecular forces involved in gel formation (Chenite et. al., 2000; Berger et. al., 2005).

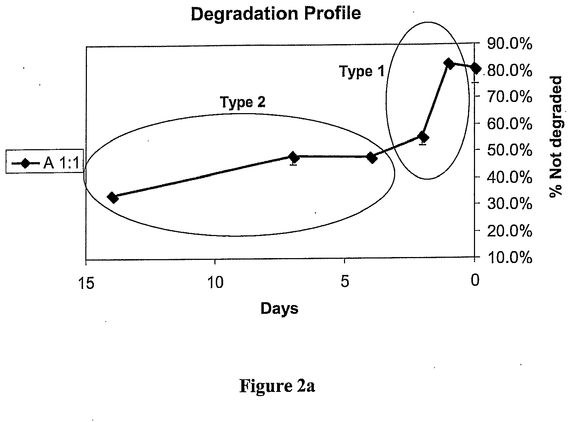

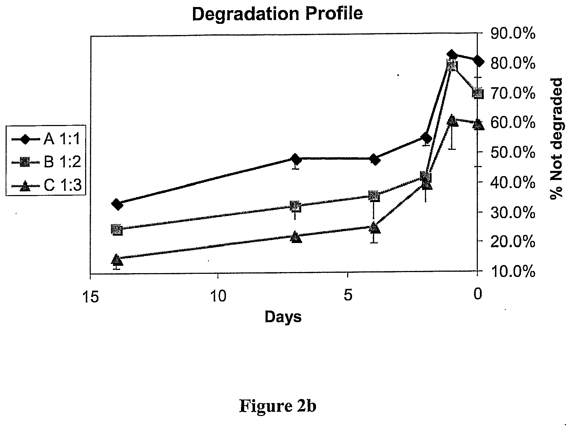

Type 1 chitosans mainly contribute to the stability, strength and rigidity of the gel, and provide slow degradation, while type 2 chitosans contribute to the softness, elasticity and fast solubilization of the gel. The different degradation profiles of type 1 and type 2 chitosans are discussed further in Example 2 below, and are shown in Figure 2.

The physical and chemical properties of the gel are altered by raising or lowering the molecular weight of the chitosans and/or their degree of acetylation, and by the natural acetylation diversity of chitosans from different sources. The properties of the gel can further be controlled by selection of the type of reacetylation (i.e. homogenous or non-homogenous), or by mapping the patterns of distribution of the deacetylated/acetylated sites.

Preferably, the highly acetylated chitosan is homogenously acetylated. Further preferably, the highly deacetylated chitosan is non-homogenously deacetylated.

Increasing the molecular weight of the chitosan increases its viscosity, such that the polymer is highly hydrated and highly hydrophobic. A gel formed from such a polymer therefore has an increased strength, and greater water retention. This results in a slow degradation rate, slow drug release, and improved mechanical properties. Preferably, each of the highly acetylated and highly deacetylated chitosans have a molecular weight of from about 10 kDa to about 4000 kDa. Molecular weight of chitosan may be easily determined by size exclusion chromatography as reported for example by O. Felt, P. Purrer, J. M. Mayer, B. Plazonnet, P. Burri and R. Gurny in Int. J. Pharm. 180, 185- 193 (1999). The upper limit of MW is determined by the ease of administration, which depends on the chosen application.

Increasing the degree of acetylation results in increased hydrophobicity in the range of 0-30% DA, but at higher values, such as 30-60% DA, the polymer begins to become more and more soluble as the amount of DA is increased. Furthermore, increasing the number of acetyl glucosamine groups increases the rate of degradation in the body, due to increased recognition sites for lysozyme. Hence, the rate of release of the hydrogel from the body can be controlled by varying the degree of chitosan acetylation.

Variations in the molecular weight, degree of deacetylation and the distribution of the acetylated sites affects the conditions (pH, temperature etc.) under which gel formation occurs; solubility; biodegradability; degree of reactivity with proteins, active pharmaceutical ingredients or other chemicals; hydrophobicity/hydrophilicity; degree of hydration; as well as biological and biocompatibility properties of the gel, such as effect on cell growth, proliferation and survival, ability of chitosans to function as inflammatory or anti-inflammatory mediators, and the effect of chitosans on acceleration or deceleration of wound healing.

For example, Type 1 chitosans of higher molecular weight have higher hydrophobicity and higher viscosity, resulting in a stronger gel due to higher intermolecular interactions. Type 1 chitosans of higher DDA have a lower rate of degradation. Type 1 chitosans having higher crystallinity have a lower degradation rate due to the fact that the crystalline form is non-soluble. Hence one skilled in the art can predict properties of the resultant gel mixture, and would therefore be able to create gels having desired characteristics, using unique combinations of the different types of chitosan.

Preferably, each of the highly acetylated and the highly deacetylated chitosans are present at a concentration of about 0.1% to 6% w/v of the total composition.

The composition of the present invention forms a gel at body temperature and physiological pH. The gel of the present invention does not require glycerophosphate.

The hydrogel of the present invention offers greater possibility of controlling gel strength, rate of degradation, and release rate than the Chitosan/βGP based hydrogel patented by BioSyntech, and extends the possibilities of controlling the gel's properties, and tailoring them to the needs of a much wider range of chemical and physical uses.

The polysaccharide hydrogel according to the present invention comprises a hybrid of chitosan with a negatively charged polysaccharide, such as a glycosaminoglycan, for example, hyaluronic acid or chondroitin sulphate.

Addition of hyaluronan has been found to cause precipitation of the composition comprising type 1 and type 2 chitosans to form a stable gel.

The hydrogel of the present invention may further comprise a third chitosan, selected from either type 1 or type 2, having a different molecular weight or degree of deacetylation, thus extending control over the resultant hydrogel.

Thus, different compositions and mixtures based on these two types of chitosans may be used to provide semi-solid gels with suitable properties for a wide range of applications, such as drug or protein delivery systems e.g. for slow release of agents or medications, scaffolding of various consistencies, including gels for supporting cell growth or bone structural support; cartilage repair; tissue reconstruction; in wound-dressings, promoting scar free healing and macrophage activation; for production of artificial skin; as a hipolipidemic and hipocholesterolimic agent; as an artificial kidney membrane; for bone filling; and heel pain relief.

The gel may be formed in situ sub-cutaneously, intra-peritoneally, intramuscularly or within biological connective tissues, bone defects, fractures, articular cavities, body conduits or cavities, eye cul-de-sac, or solid tumors.

The polysaccharide gel solution may be introduced within an animal or human body by injection or endoscopic administration,

Drugs, polypeptides, living microorganisms, animal or human cells may be incorporated within the polysaccharide gel prior to gelation.

In accordance with the present invention there is also provided the use of the polysaccharide gel for producing biocompatible degradable materials used in cosmetics, pharmacology, medicine and/or surgery.

The gel may be incorporated as a whole, or as a component, into implantable devices or implants for repair, reconstruction and/or replacement of tissues and/or organs, either in animals or humans.

The gel may be used as a whole, or as a component of, implantable, transdermal or dermatological drug delivery systems.

The gel may be used as a whole, or as a component of, opthalmological implants or drug delivery systems.

The gel may be used for producing cells-loaded artificial matrices that are applied to the engineering and culture of bioengineered hybrid materials and tissue equivalents.

The loaded cells may be selected from the group consisting of chondrocytes (articular cartilage), fibrochondrocytes (meniscus), ligament fibroblasts (ligament), skin fibroblasts (skin), tenocytes (tendons), myofibroblasts (muscle), mesenchymal stem cells, keratinocytes (skin), and neurons, as well as adipocytes or bone marrow cells. In fact cells from any tissue which are capable of proliferation may optionally be embedded in such a construct.

A major detriment to wound heeling is the presence of biofilm. Biofilm is composed of at least 80 percent extracellular macromolecules that are usually positively charged, similar to chitosan. Chitosan may optionally be used as a biofilm disruptor thus helping wound hygiene and limiting the inhibitory effect of biofilm on destruction of bacteria. Chitosan gel mixed with lactoferrin may optionally act as a slow release reservoir to destroy biofilm in any chronic wound or a wound that may become chronic. Chitosan gel mixed with xylitol may optionally also be a specific biofilm disruptor.

In accordance with the present invention there is also provided the use of loaded polysaccharide gel as injectable or implantable gel biomaterials which act as supports, carriers, reconstructive devices or substitutes for the formation in situ of bone-like, fibrocartilage-like or cartilage-like tissues at a physiological location of an animal or a human.

For example, chitosan gels according to the present invention may be useful as a sustained delivery drug-system for treatment of the eye. Results based on the ocular irritation test of chitosan compounds have indicated that chitosan preparations are suitable for use as ophthalmic gels based on their excellent tolerance (Molinaro et. al., 2002).

In accordance with a further embodiment of the present invention, a slow release drug delivery hydrogel system is provided comprising highly acetylated type 1 chitosans and highly deacetylated type 2 chitosans.
Any of the drug delivery systems of the present invention may be used for delivery of a wide variety of drugs, including, but not limited to, analgesics, anesthetics, antiacne agents, antiaging agents, antibacterials, antibiotics, antiburn agents, antidepressants, antidermatitis agents, antiedemics, antihistamines, antihelminths, antihyperkeratolyte agents, antiinflammatory agents, antiirritants, antilipemics, antimicrobials, antimycotics, antioxidants, antipruritics, antipsoriatic agents, antirosacea agents antiseborrheic agents, antiseptics, antiswelling agents, antiviral agents, antiyeast agents, cardiovascular agents, chemotherapeutic agents, corticosteroids, fungicides, hormones, hydroxyacids, keratolytic agents, lactams, mitocides, non-steroidal anti-inflammatory agents, pediculicides, progestins, sanatives, scabicides, and vasodilators.

In accordance with a further embodiment of the present invention, a method is provided for the production of a stable hydrogel comprising a composition of at least one highly acetylated chitosan having a degree of acetylation of from about 30 to about 60%, and at least one highly deacetylated chitosan having a degree of deactylation of from about 70%. The method comprises the steps of dissolving a highly acetylated chitosan in HCl solution; dissolving a highly deacetylated chitosan in HCl solution; mixing the solution of highly acetylated chitosan with the solution of highly deacetylated chitosan to form a composite solution; adjusting the pH of the composite solution to a neutral pH; and increasing the temperature of the composite solution to about 37°C.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

As used herein the term "about" refers to ± 10 %.

As used herein, the term "pseudo- thermosetting" in connection with the composition of the present invention means that temperature does not induce the gelation of the composition but acts rather as a catalyst which dramatically shortens the gelation time when risen.

As used herein, the term "neutralized" means a pH of 6.8-7.2.

### EXAMPLES

### Example 1: preparation of chitosan hydrogel

Chitosan 3% with a degree of acetylation of 15% and molecular weight of 65 KDa (Koyo, Japan) was dissolved by mixing with 0.9% HCl for 24 hours, forming a type 1 chitosan solution.

Three percent homogenously deacetylated chitosan with 51% deacetylation and molecular weight of 220 KDa (Koyo, Japan) was dissolved by mixing with 0.9% HCl for 24 hours, forming a type 1 chitosan solution

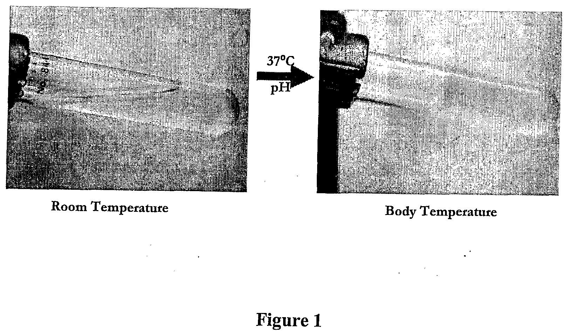

The type I and type 2 chitosan solutions were mixed according to the following ratios of type-1 to type-2: 1:1, 2:1 and 3:1, titrated to pH 6.8 and left for 24 hours at 4°C, followed by further titration to pH 7.2 at 4°C with sodium hydroxide. The resulting composition was liquid at room temperature. Upon increasing the temperature to 37°C and raising the pH to 7.4, the liquid solution formed a stable semi-solid gel, as illustrated in Figure 1.

One gram of the gel was placed in 50 ml plastic tubes, in triplicate.

Aliquots of 3 ml of 10% bovine serum media were added to each tube for predefined times intervals (1, 2, 3, 4, 5, 6, and 7 days). At the end of each time interval, the gel was washed 3 times over a period of 24 hours by repeatedly adding 50 ml of distilled water, leaving at room temperature for a few hours and removing the water. The washing process removed all soluble materials from the gel.

The gel was then frozen, lyophilized and weighed. Weight degradation was calculated from the change in weight of the samples, as a function of time interval, as is shown in Fig 2a.

### Example 2: Degradation times of chiosan gels

Pseudo-thermosetting hydrogel (3%) was prepared at a ratio of 1:1 w/w of homogenous (type 2) to non-homogenous chitosan (type 1). The degradation of the hydrogel by serum enzymes is shown in Figure 2a and 2b.

Degradation was measured at 2, 4, 7 and 14 days, by loss of gel weight.

Two distinct types of degradation kinetics are shown in Figure 2a, a fast phase that terminates within 3-6 days and a slower one that exhibits only partial degradation after 14 days. It is believed that the fast phase reflects degradation of type 2 chitosan, which is highly soluble and readily recognized by serum enzymes. The slow kinetic phase is related to chitosan type 1 chitosan, which is not readily recognized and digested by serum enzymes.

Controlling the reacetylation of glucosamine polymer is a very important tool for manipulating the extent of recognition of the chitosan by lysozyme and consequently for manipulating the rate of hydrogel degradation. The main factor that controls the activity of the enzyme is the percentage of N-acetyl glucosamine (NAG) in the polymer (Ran et al 2005). For this reason decreasing the reacetylation degree from 50% to 35% in chitosan Type 2 should allow the rate of degradation to be significantly decreased, resulting in a much shallower slope (Fig 2). On the other hand, increasing the degree of acetylation of chitosan Type 1 results in faster degradation of the polymer (Fig 2). Selection of the appropriate combination of the two types of chitosans is expected to result in a single, linear degradation curve over time, instead of the two slopes shown in this Figure.

Reference is now made to Figure 2b that illustrates mixtures of type 1 and type 2 chitosans in ratios of type 1 to type 2 of 1: 1, 2:1 and 3:1. As shown in the Figure, the rate of degradation of the gel is increased with increasing ratios of type 2 chitosan to type 1.

### Example 3: Slow release of proteins by chitosan gels

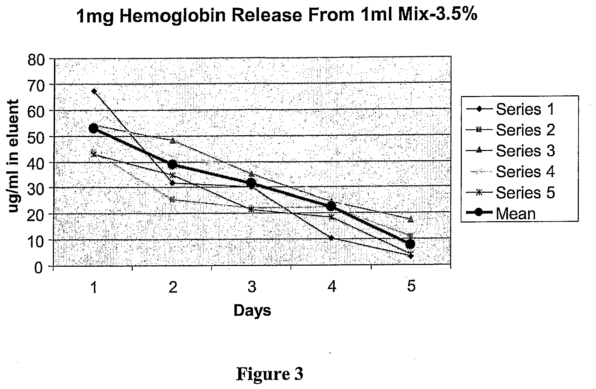

In order to study the potential of the chitosan pseudo thermosetting hydrogel of the present invention as a slow release vehicle, hemoglobin and bovine serum albumin (BSA) were used as solutes. These compounds are well accepted as protein standards. To one ml solution containing the chitosan mixture, a 25 µl aliquot of BSA or 40 µl of hemoglobin were added, resulting in a final concentration of 1 mg/ml and 4 mg/ml protein in the hydrogel, respectively. The proteins were incubated in 3 ml PBS for one week at 37°C. The media was replaced daily, and the amounts of the released protein from the hydrogel, was measured as shown in Figures 3-7.

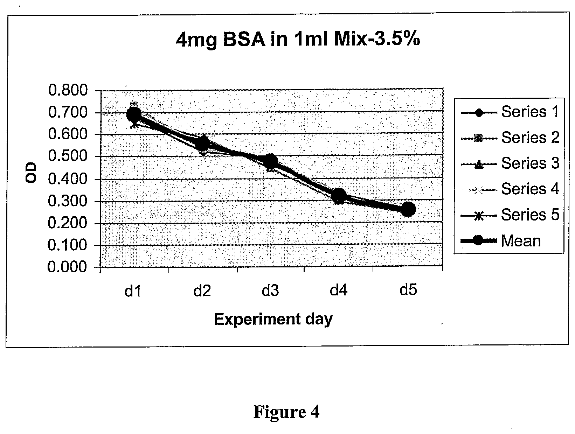

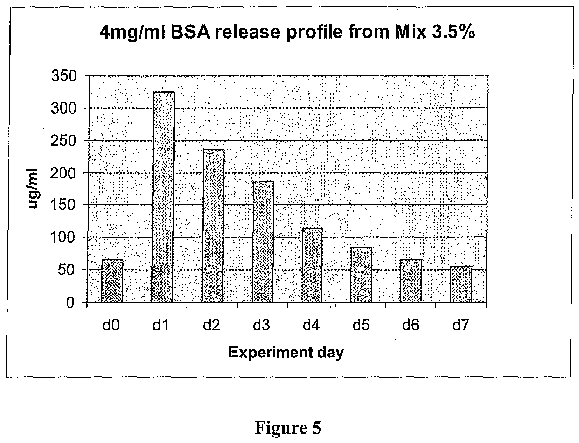

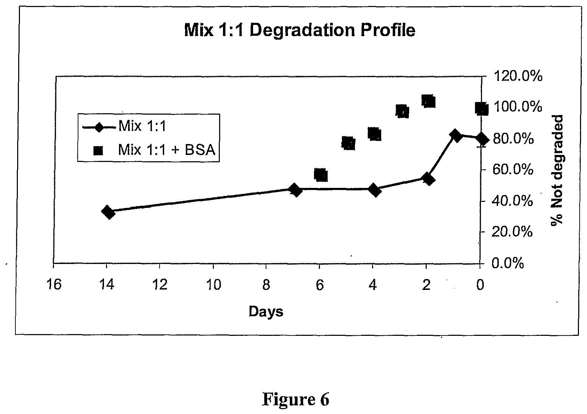

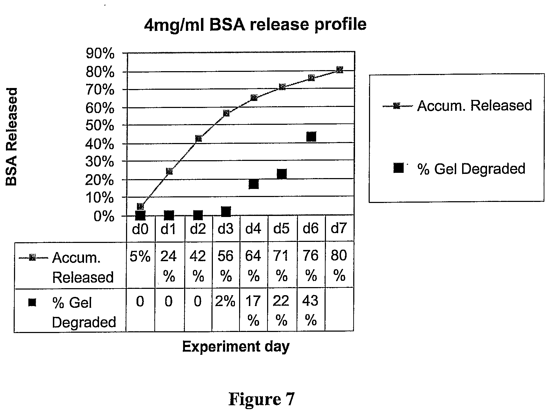

High amounts of hemoglobin were initially released and the rate of release decreased with time (Figure 3). No initial burst was shown. BSA showed the same profile as hemoglobin (Figures 4 and 5). A near linear slope was obtained (Figure 4). Mixing of the BSA with the gel improved the gel's stability, providing a decreased degradation rate compared to that of the chitosan mixture alone (Figure 6). Comparison of the amounts of the released BSA versus the amounts of the degraded gel (Figure 7) showed that the rate of release of the protein was faster than the rate of gel degradation.

The data shown in Figure 7 relate to a single degree of acetylation of type 1 chitosan and a single molecular weight, which resulted in a protein release profile having a rate of release which decreased each day. However, appropriate selection of additional variables such as degree of reacetylation and molecular weights of the two types of chitosans allows the characteristics of the gel to be determined, and enables affinity of the protein drug for the chitosan structure to be improved. Such specific combinations would be expected to provide a fixed rate of release of a specific drug, reflecting a combined diffusion and matrix degradation rate.

### Example 4: In vivo study of chitosan gel as wound dressing

Psammomys obesus strain rats, which are known to develop diabetic symptoms when raised in captivity on a high fat diet, were used as a model of type II Diabetes mellitus. These animals are considered to be an excellent model for simulating chronic skin ulcers of diabetics, and study of skin wound healing, due to their tendency to develop profound infections, gangrene and sepsis, leading to morbidity and even mortality.

The following are the common parameters for examining skin ulcers healing:
1. Timing of neovascularization appearance in the reparative tissue.
2. Reduction in neutrophil activity.
3. Accelerated macrophage activity
4. Timing of scar wound closure by a complete re-epithelialization of the wound.
5. Formation of keratinocytes monolayers.
6. Binding of the epidermis and the dermis layers by activation of the fibroblast -depositing extracellular matrix network.

A chitosan-based gel, serving as a biological dressing, was used, avoiding the need for bandaging or suturing, and providing direct coating of the wound bed for enhanced healing. The rate at which various healing stages occurred, especially the wound contraction-scar shrinkage and closure stage, was studied over a period of eight days,

Twenty five female Psammomys obesus rats of mature age, each weighing 150-160 grams were used.

Thirteen animals were found to have developed diabetes following administration of a high fat diet, starting from 4 to 6 weeks prior to day zero.

Six animals having normal euglycemia (normoglycemia), indicating resistance to development of upon feeding with a high fat diet, were used as a first control, and six animals with normoglycemia when fed on a normal low fat, low energy diet, were used as a second control.

At day zero, a round full depth punch biopsy of 6 mm diameter was made through the epidermis, dermis and hypodermis to the muscles, at the shaved skin of the neck, using a Metricoconventer-production device.

The injuries of seven diabetic animals were treated by administration of the chitosan based-gel of Example 1 to the wound area, while a further six animals were left untreated. The gel was reapplied to the wound area of the treatment group every day.

For a period of seven days all the animals were macro-photographed, and the dimensions of the wound measured every 3 days. Weight and blood glucose levels were measured once a week, using digital glucometer, (Ascensia Elite of Bayer), by absorbing a blood drop from a cut created at the tail of the rat.

After 7 days, the animals were sacrificed and full depth biopsies were performed. The skin was collected and placed in fixation solution. Skin samples were further processed for histological and immuno-histochemical staining procedures, to evaluate the differences between treated and untreated wounds.

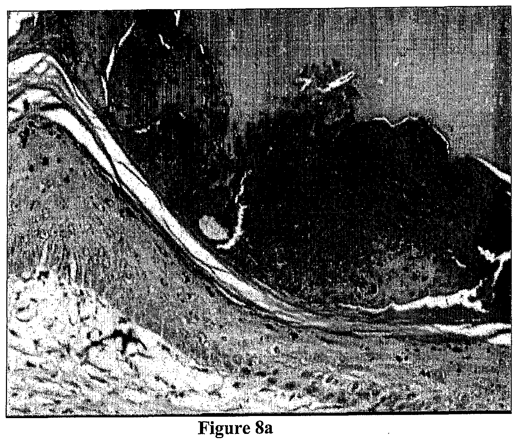

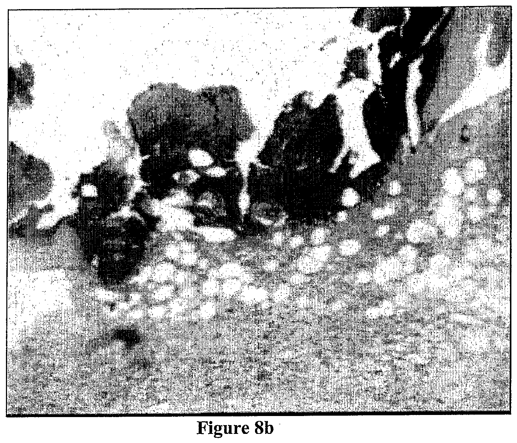

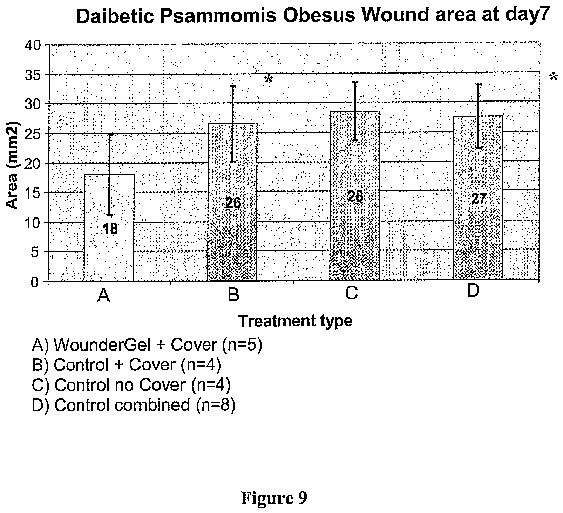

Results are shown in Figures 8a and 8b and Figure 9. As shown in the Figures, the treatment group showed a statistically significant increase in wound healing, and a reduction of the period of time required for wound healing, compared to the control group.

### Example 5 - Rotator cuff repair

Rotator cuff tears are a common source of shoulder pain. The incidence of rotator cuff damage increases with age and is most frequently caused by degeneration of the tendon, rather than injury from sports or trauma. Treatment recommendations vary from rehabilitation to surgical repair of the torn tendon(s). The best method of treatment is different for every patient and indeed many patients do not achieve satisfactory repair of their injuries.

The present invention, in some embodiments, overcomes these drawbacks of the background art by providing an injectable product allowing delivery of autologous cells into rotator-cuff tears under ultrasonographic control. In other embodiments, the injectable product allows the incorporation of bone-marrow cells as well, for example for tissue healing.

Preferably, the procedure is performed as an outpatient procedure or an ambulatory procedure requiring local anesthesia.

The initial liquid property of the gel allows full adherence to the tendon tear area.

In vivo experiments were performed on rats for tendon damage and repair (using surgically damaged tendons). The damaged tendons were sutured and were treated with a mixture of a gel according to the present invention with bone marrow cells; the control animals only received sutures. 20 animals were studied for 3 months. Histologically proven tendon repair and prevention of muscle atrophy were both achieved (results not shown).

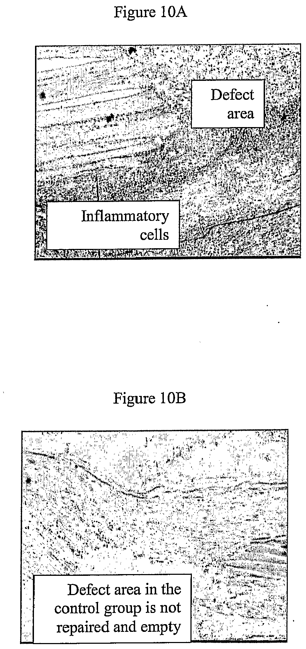

Also in vivo experiments were performed on rats for rotator cuff damage, again surgically induced. This damage was treated as above. Histological slices of tissue, 6 weeks post surgery, show that endogenous cells were trapped from the neighboring tissues, improving the status of the injured site, compared to non treated control defects in the contra lateral shoulder. Exemplary results are shown in Figure 10A (treated) and Figure 10B (non-treated).

### Bibliography

Aiba, 1991: Int. J. Biol. Macromol. 13: 40-44.
Aiba, 1994: Carbohydr. Res. 261: 297-306.
Berger et al., 2004: Int. J. Pharmaceutics 288: 197-206
Biagini et. al., 1992: in: C.J. Brine, P.A. Sandford, J.P. Zikakis (Eds.). Advances in
Chitin and Chitosan; Elsevier Science, Barking; 1: 16-24
Calvo et. al., 1997: Int. J. Pharm. 153: 41-50.
Chenite et al., 2000: Biomaterials 21: 2155-2161
Felt et. al., 1999: Int. J. Pharm.180: 185-193.
Felt et. al., 2000: J. Ocul. Pharmacol. Ther. 16: 261-270
He et. al., 1998: Int. J. Pharm. 166: 75-88.
Hoemann et al., 2005: J. Bone Joint Surg. Am 87: 2671-2686
Junginger and Verhoef, 1998: PSTT 370-376
Katsube et al., 2000: Arch. Orthop. Trauma Surg. 120: 121-127
Koga 1998: Adv. Chitin Sci. 3: 16-23.
Kotze et. al., 1999: in: E. Mathiowitz, D.E. Chickering III, C.M. Lehr (Eds.).
Bioadhesive Drug Delivery Systems, Marcel Dekker Inc. New York, 341-385.
Lee et al., 2006: Smart Mater. Struct. 15: 607-611
Liu et. al., 2001: J. Appl. Polym. Sci. 79:1324-1335
Mi et al., 2000: J. Polym. Sci. A: Polym. Chem. 38: 2804-2814
Milot et. al., 1998: J. Appl. Polymer. Sci. 68: 571-580.
Molinaro et. al., 2002: Biomaterials 23: 2717-2722.
Muzzarelli, 1986: In: Muzzarelli, R.A.A., Jeuniaux, C., Gooday, G.W. (Eds.), The
Determination of the Degree of Acetylation of Chitosans by Spectrophotometry.
Plenum Press, New York, (1986) 385-388.
Muzzarelli 1997: Cell Mol. Life Sci. 53: 131-140
Ogawa and Yui, 1993: Biosci. Biotechol. Biochem. 57: 1466-1469.
Patashnik et. al.; 1997: J. Drug Target 4: 371-380
Roughley et al., 2006: Biomaterials 27: 388-396
Song et. al., 2001: J. Nucl. Med. 28: 489-497
Ueno et. al., 2001: Adv. Drug Delivery Rev. 52: 105-115

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.

## Claims

1. A chitosan composition comprising at least one type of chitosan having a degree of acetylation in the range of from 30% to 60%, and at least one type of chitosan having a degree of deacetylation of at least 70%, wherein the composition further comprises a negatively charged polysaccharide, and wherein the composition forms a hydrogel at near physiological pH and at 37°C.

2. The composition of claim 1,
wherein said highly acetylated chitosan is either homogenously acetylated or homogenously deacetylated; or
wherein said highly deacetylated chitosan is non- homogenously deacetylated; or wherein said highly acetylated chitosan and said highly deacetylated chitosan are each present at a concentration of from about 0.2% to about 3% w/v of the total composition; or
wherein said highly acetylated chitosan and said highly deacetylated chitosan each has a molecular weight in the range of from about 10 kDa to about 4000 kDa, preferably wherein said highly deacetylated chitosan has a molecular weight of greater than about 60 kDa, more preferably wherein said highly acetylated chitosan has a molecular weight of greater than about 200 kDa; or
wherein said negatively charged polysaccharide is selected from the group consisting of an animal-derived polymer and a plant- derived polymer, more preferably wherein said plant-derived polymer is derived from seaweed; or alternatively preferably wherein said negatively charged polysaccharide is a glycosaminoglycan, more preferably wherein said glycosaminoglycan is selected from the group consisting of hyaluronic acid, chondroitin sulfate, keratane sulphate and other negatively charged polymers like dextran sulphate; or
further comprising one of a drug, polypeptide, and a cell, preferably wherein said cell is selected from the group consisting of a human cell and an animal cell, or wherein said drug is selected from the group consisting of analgesics, anesthetics, antiacne agents, antiaging agents, antibacterials, antibiotics, antiburn agents, antidepressants, antidermatitis agents, antiedemics, antihistamines, antihelminths, antihyperkeratolyte agents, antiinflammatory agents, antiirritants, antilipemics, antimicrobials, antimycotics, antioxidants, antipruritics, antipsoriatic agents, antirosacea agents antiseborrheic agents, antiseptics, antiswelling agents, antiviral agents, antiyeast agents, cardiovascular agents, chemo therapeutic agents, corticosteroids, fungicides, hormones, hydroxyacids, keratolytic agents, lactams, mitocides, non-steroidal anti-inflammatory agents, pediculicides, progestins, sanatives, scabicides, and vasodilators; or
wherein the properties of said hydrogel are controlled by manipulation of said highly acetylated chitosan and said highly deacetylated chitosan, preferably
wherein said properties of said hydrogel controlled by said manipulation are selected from the group consisting of gelation temperature, density and porosity, or
wherein said manipulation comprises manipulation of a feature selected from the group consisting of degree of hydration and degree of hydrophobicity; or
wherein degradation rate of said hydrogel is controlled by binding of the lysozyme inhibitor Tri-N-acetyl-glucosamine to said highly acetylated chitosan; or
further comprising an emulsifier, preferably wherein said chitosans and said emulsifier form nanoparticles.

3. A method for the production of the stable hydrogel of claim 1, the method comprising:
a) dissolving one type of chitosan having a degree of acetylation in the range from 30% to 60% and at least one type of chitosan having a degree of deacetylation of at least 70% in acid to form a composite solution;
b) adjusting the pH of said composite solution to a value of from 6.6 to 7; and
c) increasing the temperature of said composite solution to 37°C while raising the pH to at least 7.0.

4. The method of claim 3,
wherein said dissolving of said highly acetylated chitosan and said highly deacetylated chitosan is performed substantially simultaneously in the same vessel; or
wherein said dissolving of said highly acetylated chitosan and said highly deacetylated chitosan is performed in separate vessels to form two solutions, and wherein the method further comprises the step of mixing said two solutions to form a composite solution.

5. Hydrogel of claim 1 for use in an application selected from the group consisting of drug delivery, support of cell growth, bone structural support, cartilage repair, tissue reconstruction, wound-healing, production of artificial skin, hypolipidemic agent, hypocholesterolimic agent, formation of artificial kidney membrane, bone filling, and soft tissue reconstruction,
preferably wherein said composition is administered by a route selected from the group consisting of direct instillation, injection and endoscopic administration, or alternatively preferably wherein said soft tissue reconstruction comprises providing heel pain relief.

6. Use of the hydrogel of claim 1 in the preparation of a biocompatible material for use in the preparation of an implantable device, preferably wherein said implantable device is used for an application selected from the group consisting of tissue repair, tissue reconstruction, tissue construction, and tissue replacement.

7. Use of the hydrogel of claim 1 in the preparation of a drug delivery system, preferably wherein said drug delivery system provides slow release of an embedded medication, more preferably wherein said embedded medication is selected from the group consisting of analgesics, anesthetics, antiacne agents, antiaging agents, antibacterials, antibiotics, antiburn agents, antidepressants, antidermatitis agents, antiedemics, antihistamines, antihelminths, antihyperkeratolyte agents, antiinflammatory agents, antiirritants, antilipemics, antimicrobials, antirnycotics, antioxidants, antipruritics, antipsoriatic agents, antirosacea agents antiseborrheic agents, antiseptics, antiswelling agents, antiviral agents, antiyeast agents, cardiovascular agents, chemotherapeutic agents, corticosteroids, fungicides, hormones, hydroxyacids, keratolytic agents, lactams, mitocides, non-steroidal anti-inflammatory agents, pediculicides, progestins, sanatives, scabicides, and vasodilators, even more preferably wherein said drug delivery system is an opthalmological or urological drug delivery system.

8. Hydrogel of claim 1 for use for inhabiting invading endogenous cells in a three-dimensional gel construct; or
for embedding exogenous cells for providing metabolites, preferably wherein said metabolite is a growth factor; or
in the production of a cell-loaded artificial matrix, preferably wherein said cells are selected from the group consisting of chondrocytes, fibrochondrocytes, ligament fibroblasts, skin fibroblasts, tenocytes, myofibroblasts, mesenchymal stem cells and keratinocytes.

9. A slow release drug delivery system comprising a hydrogel comprising the composition of claim 1.

10. Slow-release drug delivery system of claim 9, for use for delivery of an agent selected from the group consisting of analgesics, anesthetics, antiacne agents, antiaging agents, antibacterials, antibiotics, antiburn agents, antidepressants, antidermatitis agents, antiedemics, antihistamines, antihelminths, antihyperkeratolyte agents, antiinflammatory agents, antiirritants, antilipemics, antimicrobials, antimycotics, antioxidants, antipruritics, antipsoriatic agents, antirosacea agents antiseborrheic agents, antiseptics, antiswelling agents, antiviral agents, antiyeast agents, cardiovascular agents, chemotherapeutic agents, corticosteroids, fungicides, hormones, hydroxyacids, keratolytic agents, lactams, mitocides, non-steroidal anti-inflammatory agents, pediculicides, progestins, sanatives, scabicides, and vasodilators; or
for delivery of a protein selected from the group consisting of BSA, and haemoglobin; or
for delivery of a non-protein agent selected from the group consisting of ACE-inhibitors and anti inflammatory drugs.

11. A chitosan composition according to claim 1, further comprising nanoparticles containing an active ingredient and encapsulated in a hydrogel.

12. The chitosan composition of claim 11, further comprising an emulsifier, preferably wherein said hydrogel forms upon injection into a subject.

13. The chitosan composition of claim 12, wherein said active ingredient comprises a protein for interacting with said nanoparticles in a non covalent interaction, said nanoparticles slowly releasing said protein in the subject upon injection.

14. Composition according to any of above claims, for use as a biofilm disruptor and optionally combined with one or more other biofilm damaging agents; or
as a carrier of one or more food additives for oral ingestion; or
as an anti adhesion device for use in the fields of surgery; or
as an injected agent for mesotherapy applications; or
as a lubricant; or
as carrier for autologous cells for reparing rotator-cuff damage and/or tendon damage.

15. A composition according to any of the above claims, further comprising autologous cells for repair of tendon damage and/or rotator cuff damage.

## Patentansprüche

1. Chitosan-Zusammensetzung, welche mindestens einen Chitosan-Typ mit einem Acetylierungsgrad im Bereich von 30% bis 60%, und mindestens einen Chitosan-Typ mit einem Deacetylierungsgrad von mindestens 70% umfasst, worin die Zusammensetzung weiter ein negativ geladenes Polysaccharid umfasst, und worin die Zusammensetzung bei annähernd physiologischem pH und bei 37°C ein Hydrogel ausbildet.

2. Zusammensetzung nach Anspruch 1,
worin das hoch-acetylierte Chitosan entweder homogen acetyliert oder homogen deacetyliert ist; oder
worin das hoch-deacetylierte Chitosan nicht homogen deacetyliert ist; oder
worin das hoch-acetylierte Chitosan und das hoch-deacetylierte Chitosan jeweils in einer Konzentration von etwa 0,2% bis etwa 3% Gew./Vol. der Gesamtzusammensetzung vorhanden sind; oder
worin das hoch-acetylierte Chitosan und das hoch-deacetylierte Chitosan jeweils ein Molekulargewicht im Bereich von etwa 10 kDa bis etwa 4000 kDa aufweisen, vorzugsweise worin das hoch-deacetylierte Chitosan ein Molekulargewicht über etwa 60 kDa aufweist, mehr bevorzugt worin das hoch-acetylierte Chitosan ein Molekulargewicht von mehr als etwa 200 kDa aufweist; oder
worin das negativ geladene Polysaccharid ausgewählt ist aus der Gruppe bestehend aus von Tieren abgeleitetem Polymer und von Pflanzen abgeleitetem Polymer, mehr bevorzugt worin das von Pflanzen abgeleitete Polymer von Seetang abgeleitet ist; oder alternativ vorzugsweise worin das negativ geladene Polysaccharid ein Glycosaminoglycan ist, mehr bevorzugt worin das Glycosaminoglycan ausgewählt ist aus der Gruppe bestehend aus Hyaluronsäure, Chondroitinsulfat, Keratansulfat und anderen negativ geladenen Polymeren, wie Dextransulfat; oder
weiter umfassend ein Arzneimittel, ein Polypeptid oder eine Zelle, vorzugsweise
worin die Zelle ausgewählt ist aus der Gruppe bestehend aus einer humanen Zelle und einer tierischen Zelle, oder worin das Arzneimittel ausgewählt ist aus der Gruppe bestehend aus Analgethika, Betäubungsmitteln, anti-Akne Mitteln, anti-aging Mitteln, antibakteriellen Mitteln, Antibiotika, antiviralen Mitteln, Antidepressiva, anti-Dermatitis-Mitteln, antiödematösen Mitteln, Antihistaminika, Antihelminthen, antihyperkeratolytischen Mitteln, anti-entzündlichen Mitteln, anti-Reiz-Mitteln, anti-lipämischen Mitteln, antimikrobiellen Mitteln, Antimycotica, Antioxidantien, juckreizstillenden Mitteln, Antipsoriatika, Mitteln gegen Rosacea, antiseborrhoischen Mitteln, Antiseptika, Mitteln gegen Anschwellen, antiviralen Mitteln, Mitteln gegen Hefen, kardiovaskulären Mitteln, chemotherapeutischen Mitteln, Corticosteroiden, Fungiziden, Hormonen, Hydroxysäuren, keratolytischen Mitteln, Lactamen, Mitociden, nicht-steroidalen anti-entzündlichen Mitteln, Pedikuloziden, Progestinen, Sanativen, Scabiciden und Vasodilatoren; oder
worin die Eigenschaften des Hydrogels durch Manipulation des hoch-acetylierten Chitosans und des hoch-deacetylierten Chitosans gesteuert wird, vorzugsweise
worin die Eigenschaften des Hydrogels durch Manipulation gesteuert werden ausgewählt aus der Gruppe bestehend aus Gelierungstemperatur, Dichte und Porösität, oder
worin die Manipulation umfasst, Manipulation eines Merkmals ausgewählt aus der Gruppe bestehend aus dem Hydratationsgrad und dem Hydrophobizitätsgrad; oder
worin die Abbaurate des Hydrogels gesteuert wird durch Binden des Lysozym-Inhibitors Tri-N-Acetylglucosamin an das hoch-acetylierte Chitosan; oder
weiter umfassend einen Emulgator, vorzugsweise worin das Chitosan und der Emulgator Nanoteilchen ausbilden.

3. Verfahren zur Herstellung des stabilen Hydrogels nach Anspruch 1, welches umfasst:
a) Lösen eines Chitosan-Typs mit einem Acetylierungsgrad im Bereich von 30% bis 60% und mindestens eines Chitosan-Typs mit einem Deacetylierungsgrad von mindestens 70% in Säure, um eine Composite-Lösung zu bilden;
b) Einstellen des pH-Wertes der Composite-Lösung auf einen Wert von 6,6 bis 7; und
c) Erhöhen der Temperatur der Composite-Lösung auf 37°C während der pH auf mindestens 7,0 erhöht wird.

4. Verfahren nach Anspruch 3,
worin das Lösen des hoch-acetylierten Chitosans und des hoch-deacetylierten Chitosans im Wesentlichen gleichzeitig in dem gleichen Behälter durchgeführt wird; oder
worin das Lösen des hoch-acetylierten Chitosans und des hoch-deacetylierten Chitosans in getrennten Behältern durchgeführt wird, um zwei Lösungen zu bilden, und worin das Verfahren weiter den Schritt umfasst, Mischen der zwei Lösungen um eine Composite-Lösung zu bilden.

5. Verwendung des Hydrogels nach Anspruch 1 in einer Anwendung ausgewählt aus der Gruppe bestehend aus Arzneimittelverabreichung, Förderung des Zellenwachstums, Förderung des Knochenaufbaus, Reparatur der Knorpel, Gewebe-Wiederaufbau, , Wund-Heilung, Produktion künstlicher Haut, hypolipidemischem Mittel, hypocholesterolimischem Mittel, Bildung künstlicher Nierenmembran, Knochenauffüllung und Wiederherstellung von weichem Gewebe,
vorzugsweise worin die Zusammensetzung über einen Weg verabreicht wird, ausgewählt aus der Gruppe bestehend aus direktem Einträufeln, Injektion und endoskopischer Verabreichung, oder alternativ vorzugsweise das die Wiederherstellung des weichen Gewebes Bereitstellen von Fersenschmerzen-Linderung umfasst.

6. Verwendung des Hydrogels nach Anspruch 1 bei der Herstellung eines biokompatiblen Materials zur Verwendung bei der Herstellung einer implantierbaren Einrichtung, vorzugsweise worin die implantierbare Einrichtung für einen Anwendung eingesetzt wird, ausgewählt aus der Gruppe bestehend aus Gewebe-Reparatur, Gewebe-Wiederherstellung, Gewebe-Konstruktion und Gewebe-Ersatz.

7. Verwendung des Hydrogels nach Anspruch 1 zur Herstellung eines Arzneimittel-Verabreichungssystems, vorzugsweise worin das Arzneimittel-Verabreichungssystem eine verzögerte Freisetzung einer enthaltenen Medikation liefert, mehr bevorzugt worin die enthaltene Medikation ausgewählt ist aus der Gruppe bestehend aus Analgetika, Betäubungsmitteln, anti-Akne Mitteln, anti-aging Mitteln, antibakteriellen Mitteln, Antibiotika, antiviralen Mitteln, Antidepressiva, anti-Dermatitis-Mitteln, antiödematösen Mitteln, Antihistaminika, Antihelminthen, antihyperkeratolytischen Mitteln, anti-entzündlichen Mitteln, anti-Reiz-Mitteln, anti-lipämischen Mitteln, antimikrobiellen Mitteln, Antimycotica, Antioxidantien, juckreizstillenden Mitteln, Antipsoriatika, Mitteln gegen Rosacea, antiseborrhoischen Mitteln, Antiseptika, Mitteln gegen Anschwellen, antiviralen Mitteln, Mitteln gegen Hefen, kardiovaskulären Mitteln, chemotherapeutischen Mitteln, Corticosteroiden, Fungiziden, Hormonen, Hydroxysäuren, keratolytischen Mitteln, Lactamen, Mitociden, nicht-steroidalen anti-entzündlichen Mitteln, Pedikuloziden, Progestinen, Sanativen, Scabiciden und Vasodilatoren; oder
sogar mehr bevorzugt, worin das Arzneimittel-Verabreichungssystem ein opthalmologisches oder urologisches Arzneimiltel-Verabreichungssystem ist.

8. Verwendung des Hydrogels nach Anspruch 1
zur Aufnahme einwandernder endogener Zellen in einem drei-dimensionalen Gel-Konstrukt; oder
zur Einbettung exogener Zellen zur Bereitstellung von Metaboliten, vorzugsweise worin der Metabolit ein Wachstumsfaktor ist; oder
bei der Herstellung einer Zell-beladenen künstlichen Matrix, vorzugsweise worin die Zellen ausgewählt sind aus der Gruppe bestehend aus Chondrozyten, Fibrochondrozyten, Ligament-Fibroblasten, Haut-Fibroblasten, Tenozyten, Myofibroblasten, mesenchymalen Stammzellen und Keratinocyten.

9. Arzneimittel-Verabreichungssystem mit verzögerter Freisetzung, welches ein Hydrogel mit der Zusammensetzung von Anspruch 1 umfasst.

10. Verwendung des Arzneimittel-Verabreichungssystem mit verzögerter Freisetzung nach Anspruch 9, zur Lieferung eines Mittels ausgewählt aus der Gruppe bestehend aus Analgethika, Betäubungsmitteln, anti-Akne Mitteln, anti-aging Mitteln, antibakteriellen Mitteln, Antibiotika, antiviralen Mitteln, Antidepressiva, anti-Dermatitis-Mitteln, antiödematösen Mitteln, Antihistaminika, Antihelminthen, antihyperkeratolytischen Mitteln, anti-entzündlichen Mitteln, anti-Reiz-Mitteln, anti-lipämischen Mitteln, antimikrobiellen Mitteln, Antimycotica, Antioxidantien, juckreizstillenden Mitteln, Antipsoriatika, Mitteln gegen Rosacea, antiseborrhoischen Mitteln, Antiseptika, Mitteln gegen Anschwellen, antiviralen Mitteln, Mitteln gegen Hefen, kardiovaskulären Mitteln, chemotherapeutischen Mitteln, Corticosteroiden, Fungiziden, Hormonen, Hydroxysäuren, keratolytischen Mitteln, Lactamen, Mitociden, nicht-steroidalen anti-entzündlichen Mitteln, Pedikuloziden, Progestinen, Sanativen, Scabiciden und Vasodilatoren; oder
zur Lieferung eines Proteins ausgewählt aus der Gruppe bestehend aus BSA, und Hämoglobin; oder
zur Lieferung eines nicht-Protein-Mittels ausgewählt aus der Gruppe bestehend aus ACE-inhibitoren und anti-entzündlichen Arzneimitteln.

11. Chitosan Zusammensetzung nach Anspruch 1, welche weiter Nanoteilchen mit einem aktiven Inhaltsstoff und eingekapselt in ein Hydrogel umfasst.

12. Chitosan-Zusammensetzung nach Anspruch 11, welche weiter einen Emulgator umfasst, vorzugsweise worin sich das Hydrogel bei Injektion in ein Individuum bildet.

13. Chitosan-Zusammensetzung nach Anspruch 12, worin der aktive Inhaltsstoff ein Protein zur Interaktion mit den Nanoteilchen in nicht-kovalenter Wechselwirkung umfasst, worin die Nanoteilchen das Protein in dem Individuum nach Injizierung verzögert freisetzen.

14. Verwendung einer Zusammensetzung nach einem der vorstehenden Ansprüche,
als Biofilm-Disruptor und wahlweise kombiniert mit ein oder mehreren anderen Biofilm-schädigenden Mitteln: oder
als Träger eines oder mehrerer Nahrungsmittel-Additive zur oralen Aufnahme; oder
als anti-Adhäsions-Einrichtung zur Verwendung im Gebiet der Chirurgie; oder
als injiziertes Mittel für Mesotherapie-Anwendungen; oder
als Schmiermittel; oder
als Träger für autologe Zellen zur Reparatur einer Rotatoren-Manschetten-Schädigung und/oder Sehnen-Schädigung.

15. Zusammensetzung nach einem der vorstehenden Ansprüche, welche weiter autologe Zellen zur Reparatur von Sehnen-Schädigungen und/oder Rotatoren-Manschetten-Schädigung umfasst.

## Revendications

1. Composition de chitosane comprenant au moins un type de chitosane présentant un degré d'acétylation dans la plage de 30% à 60% et au moins un type de chitosane présentant un degré de désacétylation d'au moins 70%, la composition comprenant en outre un polysaccharide chargé négativement et la composition formant un hydrogel à un pH proche du pH physiologique et à 37°C.

2. Composition selon la revendication 1,
ledit chitosane hautement acétylé étant, soit acétylé de façon homogène, soit désacétylé de façon homogène ; ou
ledit chitosane hautement désacétylé étant désacétylé de façon non homogène ; ou ledit chitosane hautement acétylé et ledit chitosane hautement désacétylé étant présents chacun à une concentration d'environ 0,2% à environ 3% en poids par volume de la composition totale ; ou ledit chitosane hautement acétylé et ledit chitosane hautement désacétylé présentant chacun un poids moléculaire dans la plage d'environ 10 kDa à environ 4000 kDa, ledit chitosane hautement désacétylé présentant de préférence un poids moléculaire supérieur à environ 60 kDa, ledit chitosane hautement acétylé présentant plus préférablement un poids moléculaire supérieur à environ 200 kDa ; ou
ledit polysaccharide chargé négativement étant sélectionné dans le groupe constitué par un polymère d'origine animale et un polymère d'origine végétale, plus préférablement, ledit polymère d'origine végétale étant dérivé à partir d'une algue ; ou, en variante, ledit polysaccharide chargé négativement étant de préférence un glycosaminoglycane, ledit glycosaminoglycane étant plus préférablement sélectionné dans le groupe constitué par l'acide hyaluronique, la chondroïtine sulfate, le sulfate de kératane et d'autres polymères chargés négativement, tels que le sulfate de dextran ; ou
comprenant en outre un médicament, un polypeptide et une cellule, ladite cellule étant de préférence sélectionnée dans le groupe constitué par une cellule humaine et une cellule animale, ou ledit médicament étant sélectionné dans le groupe constitué par les analgésiques, les anesthésiques, les agents antiacné, les agents antivieillissement, les antibactériens, les antibiotiques, les agents antibrûlure, les antidépresseurs, les agents antidermatite, les antioedèmes, les antihistaminiques, les anthelminthiques, les agents antihyperkératolytiques, les agents anti-inflammatoires, les anti-irritants, les antilipidémiants, les antimicrobiens, les agents antimycosiques, les antioxydants, les antiprurigineux, les antipsoriasiques, les agents anticouperose, les agents antiséborrhéiques, les antiseptiques, les agents antigonflement, les antiviraux, les agents antilevure, les agents cardio-vasculaires, les agents chimiothérapeutiques, les corticostéroïdes, les fongicides, les hormones, les hydroxyacides, les agents kératolytiques, les lactames, les acaricides, les agents anti-inflammatoires non stéroïdiens, les pédiculicides, les progestines, les agents curatifs ("sanatives"), les scabicides et les vasodilatateurs ; ou
les propriétés dudit hydrogel étant régulées par manipulation dudit chitosane hautement acétylé et dudit chitosane hautement désacétylé,
les propriétés dudit hydrogel régulées par ladite manipulation étant de préférence sélectionnées dans le groupe constitué par la température de gélification, la densité et la porosité, ou
ladite manipulation comprenant la manipulation d'une caractéristique sélectionnée dans le groupe constitué par le degré d'hydratation et le degré d'hydrophobicité ; ou
la vitesse de dégradation dudit hydrogel étant régulée par la liaison de l'inhibiteur de lysozyme tri-N-acétyl-glucosamine avec ledit chitosane hautement acétylé ; ou
comprenant en outre un émulsifiant, lesdits chitosanes et ledit émulsifiant formant de préférence des nanoparticules.

3. Procédé de production de l'hydrogel stable selon la revendication 1, le procédé comprenant :
a) la dissolution d'un type de chitosane présentant un degré d'acétylation dans une plage de 30% et 60% et d'au moins un type de chitosane présentant un degré de désacétylation d'au moins 70% dans un acide pour former une solution composite ;
b) l'ajustement du pH de ladite solution composite à une valeur de 6,6 à 7 ; et
c) l'augmentation de la température de ladite solution composite à 37°C tout en augmentant le pH à au moins 7,0.

4. Procédé selon la revendication 3,
ladite dissolution dudit chitosane hautement acétylé et dudit chitosane hautement désacétylé étant effectuée sensiblement simultanément dans le même récipient ; ou
ladite dissolution dudit chitosane hautement acétylé et dudit chitosane hautement désacétylé étant effectuée dans des récipients séparés pour former deux solutions, et le procédé comprenant en outre l'étape de mélange desdites deux solutions pour former une solution composite.

5. Hydrogel selon la revendication 1 destiné à être utilisé
dans une application sélectionnée dans le groupe comprenant
l'administration de médicament, le support de croissance cellulaire, le support de structure osseuse, la réparation du cartilage, la reconstruction tissulaire, la cicatrisation, la production de peau artificielle, un agent hypolipidémique, un agent hypocholestérolémique, la formation de membrane de rein artificiel, le remplissage d'os et la reconstruction de tissus mous,
ladite composition étant de préférence administrée par une voie sélectionnée dans le groupe constitué par l'instillation directe, l'injection et l'administration endoscopique, ou, en variante, ladite reconstruction de tissus mous comprenant de préférence l'apport d'un soulagement de la douleur du talon.

6. Utilisation de l'hydrogel selon la revendication 1 dans la préparation d'un matériau biocompatible destiné à être utilisé dans la préparation d'un dispositif implantable, ledit dispositif implantable étant de préférence utilisé pour une application sélectionnée dans le groupe constitué par la réparation tissulaire, la reconstruction tissulaire, la construction de tissu, et le remplacement de tissu.

7. Utilisation de l'hydrogel selon la revendication 1 dans la préparation d'un système d'administration de médicament, ledit système d'administration de médicament fournissant de préférence une libération lente d'un médicament incorporé, ledit médicament incorporé étant de préférence sélectionné dans le groupe constitué par les analgésiques, les anesthésiques, les agents antiacné, les agents antivieillissement, les antibactériens, les antibiotiques, les agents antibrûlure, les antidépresseurs, les agents antidermatite, les antioedèmes, les antihistaminiques, les anthelminthiques, les agents antihyperkératolytiques, les agents anti-inflammatoires, les anti-irritants, les antilipidémiants, les antimicrobiens, les antimycosiques, les antioxydants, les antiprurigineux, les antipsoriasiques, les agents anticouperose, les agents antiséborrhéiques, les antiseptiques, les agents antigonflement, les antiviraux, les agents antilevure, les agents cardio-vasculaires, les agents chimiothérapeutiques, les corticostéroïdes, les fongicides, les hormones, les hydroxyacides, les agents kératolytiques, les lactames, les acaricides, les agents anti-inflammatoires non stéroïdiens, les pédiculicides, les progestines, les agents curatifs ("sanatives"), les scabicides et les vasodilatateurs, ledit système d'administration de médicament étant même plus préférablement un système d'administration de médicaments ophtalmologiques ou urologiques.

8. Hydrogel selon la revendication 1 destiné à être utilisé
pour l'occupation de cellules endogènes envahissantes dans une construction de gel tridimensionnelle ; ou pour l'enrobage de cellules exogènes pour fournir des métabolites, ledit métabolite étant de préférence un facteur de croissance ; ou
dans la production d'une matrice artificielle chargée de cellules, lesdites cellules étant de préférence sélectionnées dans le groupe constitué par les chondrocytes, les fibrochondrocytes, les fibroblastes ligamenteux, les fibroblastes de la peau, les ténocytes, les myofibroblastes, les cellules souches mésenchymateuses et les kératinocytes.

9. Système d'administration de médicament à libération lente comprenant un hydrogel comprenant la composition selon la revendication 1.

10. Système d'administration de médicament à libération lente selon la revendication 9, destiné à être utilisé pour l'administration d'un agent sélectionné dans le groupe constitué par les analgésiques, les anesthésiques, les agents antiacné, les agents antivieillissement, les antibactériens, les antibiotiques, les agents antibrûlure, les antidépresseurs, les agents antidermatite, les antioedèmes, les antihistaminiques, les anthelminthiques, les agents antihyperkératolytiques, les agents anti-inflammatoires, les anti-irritants, les antilipidémiants, les antimicrobiens, les antimycosiques, les antioxydants, les antiprurigineux, les antipsoriasiques, les agents anticouperose, les agents antiséborrhéiques, les antiseptiques, les agents antigonflement, les antiviraux, les agents antilevure, les agents cardio-vasculaires, les agents chimiothérapeutiques, les corticostéroïdes, les fongicides, les hormones, les hydroxyacides, les agents kératolytiques, les lactames, les acaricides, les agents anti-inflammatoires non stéroïdiens, les pédiculicides, les progestines, les agents curatifs ("sanatives"), les scabicides et les vasodilatateurs ; ou
pour l'administration d'une protéine sélectionnée dans le groupe constitué par le BSA et l'hémoglobine ; ou
pour l'administration d'un agent non protéique sélectionné dans le groupe constitué par les inhibiteurs d'ACE et de médicaments anti-inflammatoires.

11. Composition de chitosane selon la revendication 1, comprenant en outre des nanoparticules contenant un ingrédient actif et encapsulé dans un hydrogel.

12. Composition de chitosane selon la revendication 11, comprenant en outre un émulsifiant, ledit hydrogel se formant de préférence après injection dans un individu.

13. Composition de chitosane selon la revendication 12, ledit ingrédient actif comprenant une protéine pour interagir avec lesdites nanoparticules dans une interaction non covalente, lesdites nanoparticules libérant lentement ladite protéine dans l'individu après injection.

14. Composition selon l'une quelconque des revendications ci-dessus, destiné à être utilisée
comme désintégrateur de biofilm et éventuellement combiné avec un ou plusieurs autres agents endommageant le biofilm ; ou
comme support d'un ou plusieurs additifs alimentaires pour ingestion orale ; ou
comme dispositif anti-adhérence destiné à être utilisé dans les domaines de la chirurgie ; ou
comme substance d'injection pour des applications de mésothérapie ; ou
comme lubrifiant ; ou
comme support pour des cellules autologues pour la réparation de lésions de la coiffe des rotateurs et/ou des lésions des tendons.

15. Composition selon l'une quelconque des revendications précédentes, comprenant en outre des cellules autologues pour la réparation de lésions des tendons et/ou des lésions de la coiffe des rotateurs.
